# EUROPEAN PATENT APPLICATION

(11) **EP 4 810 105 A1**
(43) Date of publication of application: **23.09.2026**
(21) Application number: 26164174.0
(22) Date of filing: 12.03.2026
(51) Int. Cl.: A61N 1/375, A61N 1/05, A61B 17/34, A61N 1/372

(54) **IMPLANTABLE MEDICAL DEVICE WITH DEPLOYABLE FIXATION TINES**

(30) Priority: 20.03.2025 US 202563775130 P; 11.03.2026 US 202619562992
(71) Applicant: Pacesetter, Inc., Sylmar, CA 91342 (US)
(72) Inventor: Rogan, Laura, Sylmar, CA 91342 (US); Jaconetta, Matt, Sylmar, CA 91342 (US); Zhang, Kai, Sylmar, CA 91342 (US); Chantasirivisal, Steve, Sylmar, CA 91342 (US); Alexander R, Bornzin, Sylmar, CA 91342 (US)
(74) Representative: Barker Brettell Sweden AB

(57) **Abstract**

An implantable medical device (IMD) (100) includes a housing (02), a tip electrode (104), a plurality of fixation tines (106), and a deployment mechanism (108, 300, 300a, 450, 500). The housing (102) includes a distal tip (110). The distal tip (110) has a distal face (112). The tip electrode (104) is mounted to the distal tip (110) of the housing (102) and is exposed along the distal face (112). The fixation tines (106) are mounted to the distal tip (110) of the housing (102). The fixation tines (106) include hook features (120) for securing to tissue of the patient. The deployment mechanism (108, 300, 300a, 450, 500) is able to deploy the fixation tines (106) to a deployed state. The fixation tines (106) in the deployed state are exposed along the distal face (112), and can embed into the tissue of the patient to secure the IMD (100) at a target implant location.

## Description

### TECHNICAL FIELD

Embodiments of the present disclosure relate generally to implantable medical devices (IMDs) configured to be implanted within a patient and secured to patient tissue at a target implant location.

### BACKGROUND

Some IMDs function to monitor cardiac activity of the subject, provide electrotherapy to cardiac tissue, and/or the like. Examples of IMDs include cardiac pacemakers that monitor the heart and also provide stimulation therapy by delivering electrical pacing pulses and/or shocks. Conventional pacemakers include a housing, or "can," that couples to one or more insulated wires called leads. The electrical pulses and/or shocks are generated in the housing in a portion of the patient body outside of the heart and then conveyed via the lead(s) to cardiac tissue. Leadless pacemakers can monitor the heart and deliver stimulation therapy without the use of leads.

A delivery assembly may include a guide catheter that transports the IMD into the patient, navigates the venous system and/or cardiac anatomy, and positions the IMD at a target anatomical location for implant. The IMD may be secured in place to the patient tissue at the target implant location in an attempt to prohibit creep (e.g., movement) of the IMD over time and ensure reliable and consistent stimulation therapy. Upon placement of the IMD at the target anatomical implant location, the catheter can be withdrawn from the patient while the IMD remains.

Depending on patient-specific conditions, it may be desirable to attach the IMD to a relatively thin and/or heterogenous tissue wall of the patient. For example, it may be desirable to secure the IMD to an atrial wall of the patient's heart. The atrial wall may be thinner and/or more heterogenous in composition than a ventricular wall of the patient's heart, such as the interventricular septal wall. Due to the characteristics of the thin wall at the target implant site, it may be difficult to secure the IMD via conventional fixation means at a distal end of the IMD. One conventional fixation means is four large hooks that are biased to project radially outward in different directions beyond the diameter of the IMD housing and curve towards an opposite, proximal end of the IMD. The atrial tissue may be too thin and/or heterogenous in composition to reliably and efficiently secure the IMD in place using these large hooks. Furthermore, the four large hooks have an additional drawback associated with retrieval of the IMD. Once the four large hooks are deployed into the tissue, it may be difficult to atraumatically extricate the IMD from the tissue for retrieval of the IMD from the patient, or even repositioning of the IMD at a different target implant location.

A need remains for an IMD that includes a fixation mechanism for reliably, efficiently, and atraumatically securing to various different types of patient tissue, including the relatively thin and/or heterogenous tissue of the right atrium. There is also a need for the fixation mechanism to be releasable from the tissue to permit effective IMD retrieval and repositioning.

### SUMMARY

The present invention is defined in the independent claim. Further embodiments of the invention are defined in the dependent claims.

In accordance with embodiments herein, an implantable medical device (IMD) is provided that is configured to be implanted within a patient. The IMD includes a housing, a tip electrode, a plurality of fixation tines, and a deployment mechanism. The housing includes a distal tip that has a distal face. The tip electrode is mounted to the distal tip of the housing and is exposed along the distal face. The fixation tines are mounted to the distal tip of the housing and include hook features for securing to tissue of the patient. The deployment mechanism is configured to selectively deploy the fixation tines to a deployed state. The fixation tines in the deployed state are exposed along the distal face and configured to embed into the tissue of the patient to secure the IMD at a target implant location.

Additionally, or alternatively, the tip electrode is centered along the distal face of the distal tip, and the fixation tines surround the tip electrode.

Additionally, or alternatively, the fixation tines are annularly arranged in a ring.

Additionally, or alternatively, at least some of the fixation tines are located at different radial distances from the tip electrode.

Additionally, or alternatively, the distal face of the distal tip is planar.

Additionally, or alternatively, the fixation tines in the deployed state do not outwardly extend beyond a diameter of the housing.

Additionally, or alternatively, the fixation tines are J-shaped.

Additionally, or alternatively, the plurality of fixation tines includes at least ten fixation tines.

Additionally, or alternatively, the IMD further comprises a pulse generator configured to generate electrical stimulation that is delivered, via the tip electrode, to the tissue of the patient.

In accordance with embodiments, the IMD is a leadless pacemaker.

In accordance with alternative embodiments, the IMD is a transvenous lead.

Additionally, or alternatively, the deployment mechanism is configured to selectively move the fixation tines in a distal direction relative to the distal tip of the housing from a non-deployed state to the deployed state, wherein the fixation tines in the deployed state extend beyond the distal face so that the hook features are exposed for securing to the tissue of the patient, and the hook features of the fixation tines in the non-deployed state are not exposed for securing to the tissue of the patient.

Additionally, or alternatively, the deployment mechanism is configured to selectively retract the fixation tines relative to the distal tip of the housing from the deployed state to the non-deployed state.

Additionally, or alternatively, the deployment mechanism is configured to mechanically connect to a user input device on a delivery assembly used to implant the IMD, and the deployment mechanism is configured to extend the fixation tines to different protruding distances relative to the distal face of the distal tip in the deployed state, based on varying operator input on the user input device.

Additionally, or alternatively, the deployment mechanism includes a first component and a second component that is coupled to the first component, the second component coupled to a base that holds the fixation tines, wherein the first component is configured to be rotated by the delivery assembly, and rotation of the first component causes the second component to linearly translate relative to the first component, forcing the base and the fixation tines to linearly translate relative to the distal tip of the housing.

Additionally, or alternatively, one of the first component or the second component is a threaded shaft, and the other of the first component or the second component is a nut that contains one or more ball bearings that engage a helical groove of the threaded shaft.

Additionally, or alternatively, the first component that is configured to be rotated by the delivery assembly includes an angled cam surface, the angled cam surface configured to be contacted by a distal end of a rod-like tool of the delivery assembly, wherein the first component is configured to rotate due to translation of the rod-like tool in the distal direction.

Additionally, or alternatively, the deployment mechanism is a push-actuated release system within the housing of the IMD, wherein the push-actuated release system is configured to force the fixation tines to travel in the distal direction from the non-deployed state to the deployed state in response to a first application of force exerted on the distal tip that causes the distal tip to retract in a proximal direction for at least a threshold distance.

Additionally, or alternatively, while the fixation tines are in the deployed state, the push-actuated release system is configured to force the fixation tines to retract in the proximal direction from the deployed state to the non-deployed state in response to a second application of force exerted on the distal tip that causes the distal tip to retract in the proximal direction for at least the threshold distance.

In accordance with embodiments, the deployment mechanism includes a dissolvable cap mounted to the distal tip, the dissolvable cap covering the fixation tines in a non-deployed state, the dissolvable cap composed of a biodegradable material configured to dissolve and expose the fixation tines, achieving the deployed state, after a designated period of time in contact with biological fluid within the patient.

Additionally, or alternatively, the deployment mechanism includes a dissolvable cap mounted to the distal tip, an electrical contact at a proximal end of the housing, and a wire that extends from the electrical contact to the dissolvable cap, the dissolvable cap covering the fixation tines in a non-deployed state of the fixation tines, wherein the electrical contact is configured to electrically connect to an electrical element of the delivery assembly and receive an electric current from the electrical element that is conveyed via the wire to the dissolvable cap, the dissolvable cap comprising a polymer material configured to dissolve and expose the fixation tines, achieving the deployed state of the fixation tines, in response to receiving the electric current.

In accordance with embodiments herein, a leadless pacemaker is provided that is configured to be implanted within a patient. The leadless pacemaker includes a housing, a tip electrode, a plurality of fixation tines, a pulse generator, and a deployment mechanism. The housing includes a distal tip that has a distal face. The tip electrode is mounted to the distal tip of the housing. The tip electrode is centered and exposed along the distal face. The fixation tines are mounted to the distal tip of the housing and include hook features for securing to tissue of the patient. The pulse generator is configured to generate electrical stimulation that is delivered, via the tip electrode, to the tissue of the patient. The deployment mechanism is configured to selectively deploy the fixation tines to a deployed state. The fixation tines in the deployed state are exposed along the distal face and surround the tip electrode. The fixation tines in the deployed state are configured to embed into the tissue of the patient to secure the leadless pacemaker at a target implant location.

In accordance with embodiments herein, an implantable medical device (IMD) is provided that is configured to be implanted within a patient. The IMD includes a housing, a tip electrode, a plurality of fixation tines, and a deployment mechanism. The housing includes a distal tip that has a distal face. The tip electrode is mounted to the distal tip of the housing and is exposed along the distal face. The fixation tines are mounted to the distal tip of the housing and include hook features for securing to tissue of the patient. The deployment mechanism is configured to selectively move the fixation tines in a distal direction relative to the distal tip of the housing from a non-deployed state to a deployed state. The fixation tines in the deployed state project beyond the distal face so that the hook features are exposed for embedding into the tissue of the patient to secure the IMD to the tissue. The hook features of the fixation tines in the non-deployed state do not project beyond the distal face.

### BRIEF DESCRIPTION OF THE DRAWINGS

Figure 1 is a block diagram of an IMD according to an embodiment.
Figure 2 illustrates a distal portion of the IMD with fixation tines in the deployed state according to an embodiment.
Figure 3 shows the distal portion of the IMD with the fixation tines in the non-deployed state according to an embodiment.
Figure 4 shows a leadless pacemaker according to an embodiment.
Figure 5 illustrates a sectional view of a patient's heart with the leadless pacemaker implanted therein according to an example application.
Figure 6 is a cross-sectional view of the leadless pacemaker showing a first deployment mechanism according to an embodiment.
Figure 7 is a distal view of the leadless pacemaker of Figure 6 according to an embodiment.
Figure 8 illustrates a variation of the first deployment mechanism according to an embodiment.
Figure 9 illustrates a delivery assembly that can be used to implant the IMD.
Figure 10 illustrates a cross-sectional view of a distal portion of the IMD with the fixation tines in a non-deployed state according to another embodiment.
Figure 11 illustrates a cross-sectional view of the IMD in Figure 10 with the fixation tines in the deployed state.
Figure 12 illustrates the leadless pacemaker with the fixation tines in a non-deployed state according to another embodiment.
Figure 13 illustrates a block diagram of an exemplary IMD that is configured to be implanted into the patient in accordance with embodiments herein.
Figure 14 illustrates an implantable lead that can represent the IMD with deployable fixation tines according to an embodiment.
Figure 15 is a flowchart of a method of fixating an IMD according to an embodiment to patient tissue.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

Embodiments may be implemented in connection with one or more implantable medical devices (IMDs). IMDs are devices that are implanted within the body of a patient and operate while disposed within the patient. Non-limiting examples of IMDs include neurostimulator devices, implantable leadless monitoring and/or therapy devices, catheters, and/or alternative implantable medical devices. In particulate examples, the IMD may represent a leadless pacemaker or a transvenous electrically-conductive lead of a (conventional) pacemaker. Therefore, the "IMD" referred to herein can represent, for example, a leadless pacemaker or a lead, unless specifically indicated. In an example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System". The term "leadless" shall mean an absence of transvenous and/or subcutaneous electrically-conductive leads that would otherwise traverse vessels or other anatomy inside or outside of an intra-cardiac space. The term "intra-cardiac" shall mean entirely within the heart and/or an associated vessels, including (but not limited to) such as the superior vena cava (SVC), inferior vena cava (IVC), coronary sinus (CS), pulmonary arteries, and the like.

Embodiments set forth herein include. Particular embodiments of the delivery system include an IMD that includes a housing, a tip electrode, a plurality of fixation tines, and a deployment mechanism. The tip electrode is mounted to a distal tip of the housing and is exposed along a distal face of the distal tip for physically contacting tissue of the patient. The fixation tines are mounted to the distal tip of the housing. The fixation tines have hook features for securing to the patient tissue. The deployment mechanism selectively deploys the fixation tines from a non-deployed state to a deployed state. In the deployed state, the fixation tines are exposed along the distal face and project from the distal face. When the distal end of the IMD is pressed against the patient tissue while the fixation tines are deployed, the fixation tines embed into the tissue to secure the IMD at a target implant location of the patient tissue. In an example, the fixation tines are relatively small and numerous, which collectively provide sufficient grip strength on the tissue wall to retain the IMD in place, with limited or no risk of perforating the full thickness of the tissue wall, even if the tissue wall is relatively thin and/or heterogeneous. For example, the IMD described herein could be affixed via the fixation tines to an atrial wall in the right atrium. The ability to fixate to various different kinds of tissue walls, via the fixation tines and the deployment mechanism, is beneficial for increasing stimulation therapy options for the IMD. As a non-limiting example, the IMD could be implanted in the right atrium or the right ventricle.

When the fixation tines are in the non-deployed state, the fixation tines are concealed, covered, retracted, or otherwise not exposed along the distal face. The non-deployed fixation tines may not be able to penetrate and embed into the patient tissue. In an example, an operator may control the IMD so that the deployment mechanism retains the fixation tines in the non-deployed state while the IMD is being routed through the patient to a target anatomical area and/or while the tip electrode of the IMD is being used to map the target anatomical area for determining the target implant location of the IMD. The fixation tines are non-deployed during these events to avoid the hook features snagging on patient tissue prior to determining the target implant location. Once the target implant location is determined, the deployment mechanism may deploy the fixation tines to cause the fixation tines to embed and "bite" into the patient tissue at the target implant location, securing the IMD in place.

In one or more embodiments, the fixation tines of the IMD may be retractable. Thus, the fixation tines could be retracted from the deployed state to the non-deployed state. This retractability is beneficial for IMD retrieval and repositioning. For example, while the IMD is secured to the patient tissue at the target implant location, it may be desirable to extricate the IMD from the patient tissue, either for removing the IMD from the patient entirely or repositioning the IMD at a different target implant location. The operator may interact with the deployment mechanism to retract the fixation tines, which may substantially or fully release the fixation tines from the patient tissue, allowing the operator to easily move the IMD.

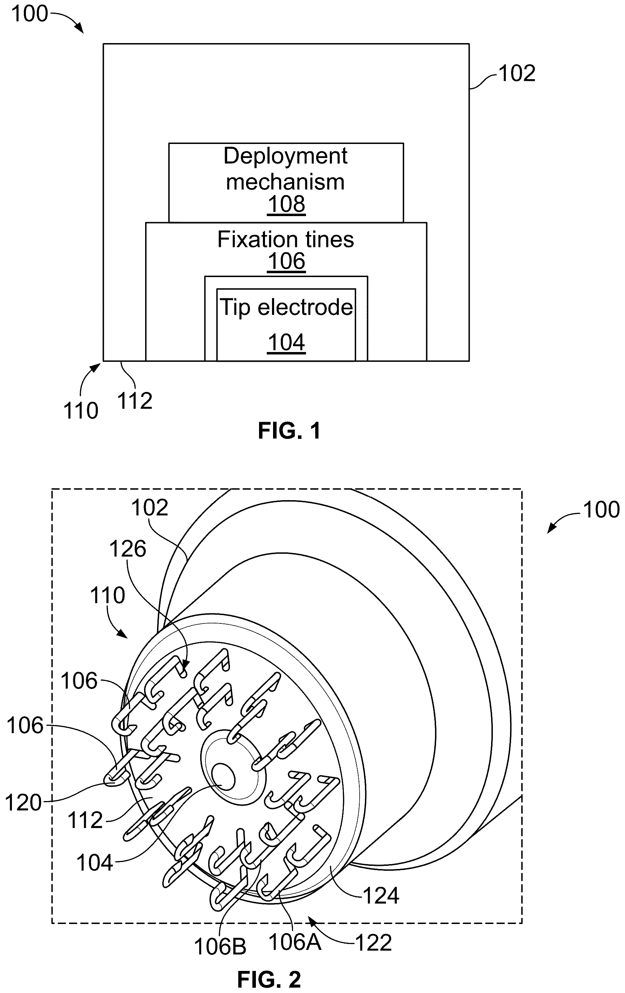

Figure 1 is a block diagram of an IMD 100 according to an embodiment. The IMD 100 includes a housing 102, a tip electrode 104, a plurality of fixation tines 106, and a deployment mechanism 108. The IMD 100 may include additional components than the components shown in Figure 1.

The housing 102 includes a distal tip 110. The distal tip 110 has a distal face 112. The tip electrode 104 is mounted to the distal tip 110, and is exposed along the distal face 112. The term "exposed" shall mean that the exposed component is unconcealed (e.g., uncovered) and open to the surrounding environment. For example, the exposed portion of the tip electrode 104 may physically contact biological fluids and tissues of the patient. The fixation tines 106 are mounted to the distal tip 110. The fixation tines 106 include hook features for securing to tissue of the patient (not shown in Figure 1). The deployment mechanism 108 is operable to selectively deploy the fixation tines 106 from a non-deployed state to the deployed state. The fixation tines 106 in the deployed state are exposed along the distal face 112 and configured to embed into the patient tissue to secure the IMD 100 at a target implant location.

When secured to the tissue at the target implant location, the IMD 100 may provide stimulation therapy to the patient, periodically or as needed. The stimulation therapy may include delivering electrical stimulation to the patient tissue via the tip electrode 104 or another electrode of the IMD 100. The electrical stimulation may be in the form of pacing pulses and/or defibrillation shocks. The defibrillation shocks may have greater energy output than the pacing pulses.

Figure 2 illustrates a distal portion of the IMD 100 with the fixation tines 106 in the deployed state according to an embodiment. The distal portion of the IMD 100 includes the distal tip 110 of the housing 102, the fixation tines 106, and the tip electrode 104. Figure 2 represents one example of the IMD 100. The subject matter described herein is not limited to the particular components and arrangement of components shown in Figure 2. The fixation tines 106 are shown in a deployed state. In the deployed state, the fixation tines 106 project beyond the distal face 112 of the distal tip 110 of the housing 102. The fixation tines 106 include hook features 120 for securing to tissue of the patient. For example, when the distal end 122 of the IMD 100 is pressed against a tissue wall of the patient with the fixation tines 106 deployed, the fixation tines 106 may penetrate tissue wall and embed into the tissue. The hook features 120 may provide grip or bite that resists separation (e.g., uncoupling) of the IMD 100 from the tissue wall. In the illustrated example, the fixation tines 106 are J-shaped, with the hook features 120 forming the curved distal portion of the J-shape. The fixation tines 106 may have other shapes in other embodiments. For example, the fixation tines 106 may have anchor-like shapes, such as with two flukes projecting in different directions from a shaft.

In an example, the tip electrode 104 may be centered along the distal face 112 of the housing 102. The tip electrode 104 may be located along a central axis of the housing 102. The tip electrode 104 may be exposed along the distal face 112 to access the patient tissue. The IMD 100 may use the tip electrode 104 during stimulation therapy for delivering electrical pulses (therapy) and/or for sensing evoked responses to delivered electrical pulses. The tip electrode 104 may have a button-shaped protrusion that slightly projects beyond the distal face 112 of the housing 102.

The plurality of fixation tines 106 may surround the tip electrode 104 along the distal face 112. For example, the fixation tines 106 may be spaced apart circumferentially around the tip electrode 104 and may be radially located between the tip electrode 104 and a perimeter edge 124 of the distal tip 110. The fixation tines 106 may be arranged in an array. The fixation tines 106 may be spaced apart from one another in the array. For example, different pairs of adjacent (e.g., neighboring) fixation tines 106 may have the same pitch or spacing between the two fixation tines 106 in the pair. In the illustrated example, the fixation tines 106 may be annularly arranged in a ring that encircles the tip electrode 104. Optionally, at least some of the fixation tines 106 are located at different radial distances from the tip electrode 104. In the illustrated example, a first subset of the fixation tines 106 including a first tine 106A forms an outer ring, and a second subset of the fixation tines 106 including a second tine 106B forms an inner ring. The inner ring is radially located between the tip electrode 104 and the outer ring. Optionally, the fixation tines 106 may be oriented so that the hook features 120 point radially inward towards the tip electrode 104. As a result, the hook features 120 of many of the fixation tines 106 point in different directions. Orienting the fixation tines 106 to point in different directions may support fixation to heterogenous patient tissue, such as a right atrial tissue wall. The fixation tines 106 may be arranged differently and/or oriented differently in other embodiments. For example, the fixation tines 106 may be arranged in a single annular ring surrounding the tip electrode 104, may be arranged in orthogonal rows and columns surrounding the tip electrode 104, and/or the like.

The fixation tines 106 may be composed of a rigid or semi-rigid material. Example materials of the fixation tines 106 may include one or more of a memory shape metal or metal alloy, a polymer, a metal, a composite material, or the like. The rigidity of the fixation tines 106 may be tailored to characteristics of the tissue at a desired implant location. For example, the rigidity may be controlled according to the type of atrial tissue to which the IMD 100 will be fixated.

The deployment mechanism 108 is at least partially contained within the housing 102 and is not visible in Figure 2. In at least one example, the deployment mechanism 108 selectively deploys the fixation tines 106 from the non-deployed position to the deployed position by advancing the fixation tines 106 in a distal direction to increase the length by which the fixation tines 106 project beyond the distal face 112. As such, the fixation tines 106 are extendable relative to the housing 102. The fixation tines 106 may be movable within small slots 126 or apertures defined through the distal face 112 of the housing 102. For example, in the non-deployed state, the hook features 120 of the fixation tines 106 may be nested within the slots 126, to avoid snagging on patient tissue.

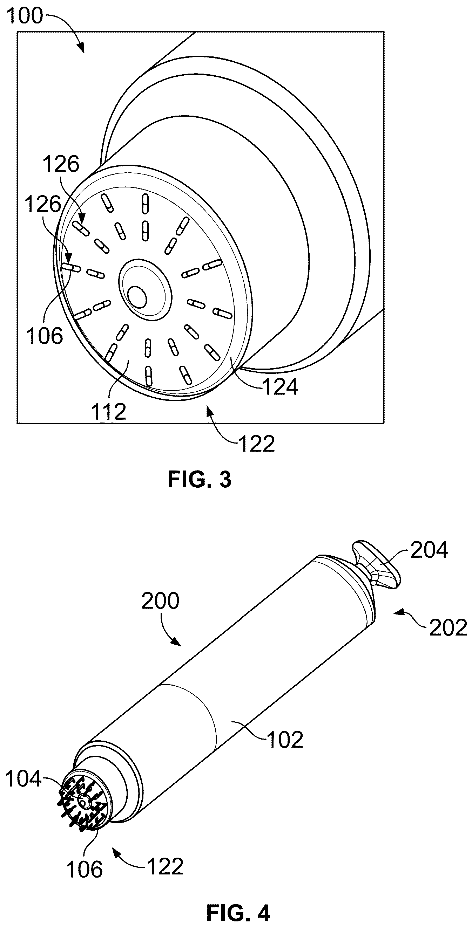

Figure 3 shows the distal portion of the IMD 100 with the fixation tines 106 in the non-deployed state according to an embodiment. The hook features 120 (shown in Figure 2) of the fixation tines 106 are not exposed for securing to the tissue of the patient. In one example, as shown in Figure 3, the hook features 120 are nested or housed within the corresponding slots 126 along the distal face 112. In another example, the hook features 120 may be covered or concealed in the non-deployed state. The fixation tines 106 may not grip, bite, embed, or otherwise secure to patient tissue as the distal end 122 is pressed against patient tissue, when in the non-deployed state.

The operator may maintain the fixation tines 106 in the non-deployed state while the IMD 100 is navigated through the patient's circulatory system towards the target implant location. The fixation tines 106 may also be in the non-deployed state for testing different candidate implant locations for the IMD 100, in a process referred to as mapping. With the fixation tines 106 retracted, Figure 3 clearly shows the dual concentric ring arrangement of the fixation tines 106. In the illustrated example, the distal face 112 of the housing 102 is planar or approximately planar. In another example, the distal face 112 may be curved, such as with a convex curve. For example, the distal face 112 may taper in a proximal direction with increasing radial distance from the tip electrode 104 to the perimeter edge 124. In at least one example, the deployment mechanism 108 may be able to selectively extend and retract the fixation tines 106 to repeatedly transition between the deployed and non-deployed states.

Referring back to Figure 2, the fixation tines 106 have substantially different characteristics and operation than the four large hooks of the conventional fixation means described in the background. For example, instead of a limited number of large hooks, the IMD 100 described herein has a relatively large number of small fixation tines 106. The IMD 100 may include at least ten fixation tines 106. In an example, the IMD 100 includes at least twenty fixation tines 106 at the distal end 122. Furthermore, the fixation tines 106 are relatively small in length and reach. When in the deployed state, each fixation tine 106 may project a length from the distal face 112 that is no greater than 6 mm. In an example, the lengths of the deployed fixation tines 106 may be no greater than 4 mm, or no greater than 2 mm. This limited length allows for shallower fixation to the tissue wall, permitting the IMD 100 to be secured to relatively thin patient walls (e.g., thin right atrial tissue). Furthermore, the radial or lateral reach of the fixation tines 106 is generally confined to the area of the distal face 112. In an example, the fixation tines 106 in the deployed state do not radially outwardly extend beyond a diameter of the housing 102. In the illustrated example, the fixation tines 106 do not overlap or radially extend beyond the perimeter edge 124.

The IMD 100 shown in Figures 2 and 3 may be one of various different types of IMDs. For example, the distal portion shown in Figures 2 and 3 may be a distal portion of a transvenous lead that represents the IMD 100. A proximal end of the transvenous lead may couple to a pacemaker device. The transvenous lead may be for cardiac stimulation or neurostimulation. In another example, the distal portion shown in Figures 2 and 3 is a distal portion of a leadless pacemaker.

Figure 4 shows a leadless pacemaker 200 according to an embodiment. The leadless pacemaker 200 may represent the IMD 100 described herein. For example, the leadless pacemaker 200 is an intra-cardiac medical device (LIMD). The leadless pacemaker 200 includes the fixation tines 106, the tip electrode 104, the housing 102, and the deployment mechanism 108. The deployment mechanism 108 may be concealed within the housing 102. The housing 102 may have a cylindrical shape. The housing 102 extends from a proximal end 202 of the housing 102 to the distal end 122. The leadless pacemaker 200 may have a horn 204 that projects in a proximal direction from the proximal end 202 of the housing 102. The horn 204 may be used to couple to the leadless pacemaker 200 during implant, positioning, and/or retrieval. For example, a tether line may lasso the horn 204 to pull the leadless pacemaker 200 during retrieval and/or repositioning operations.

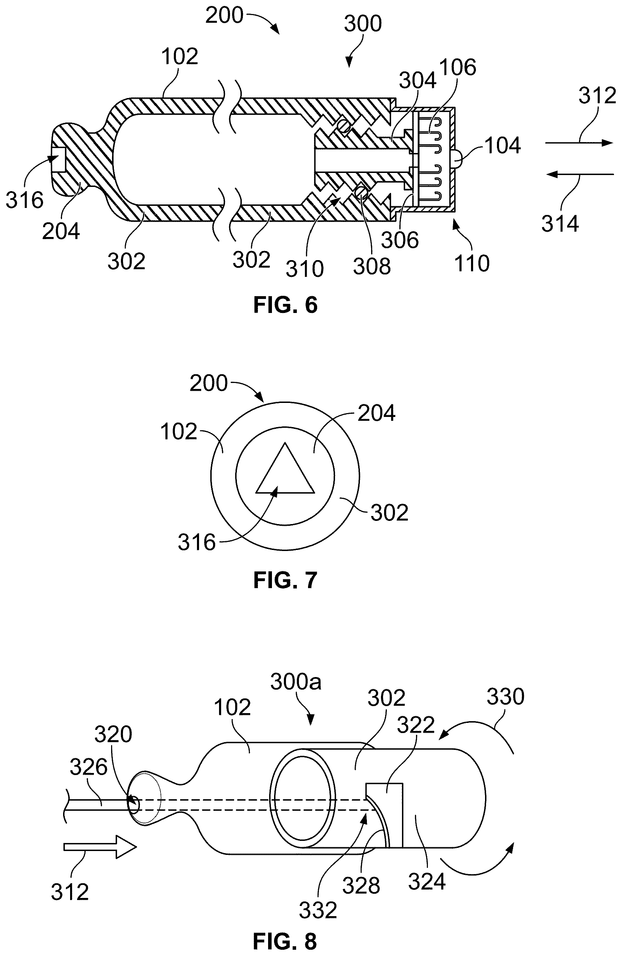

The housing 102 may contain a controller, a battery, sensing circuitry, a pulse generator, and/or the like. The leadless pacemaker 200 may have one or more additional electrodes besides the tip electrode 104. For example, the housing 102, or a portion thereof, may be electrically conductive and may function as an electrode. In another example, the leadless pacemaker 200 may include a ring electrode that is located proximal to the tip electrode 104. The components of the leadless pacemaker 200 may be described in more detail with reference to Figure 6.

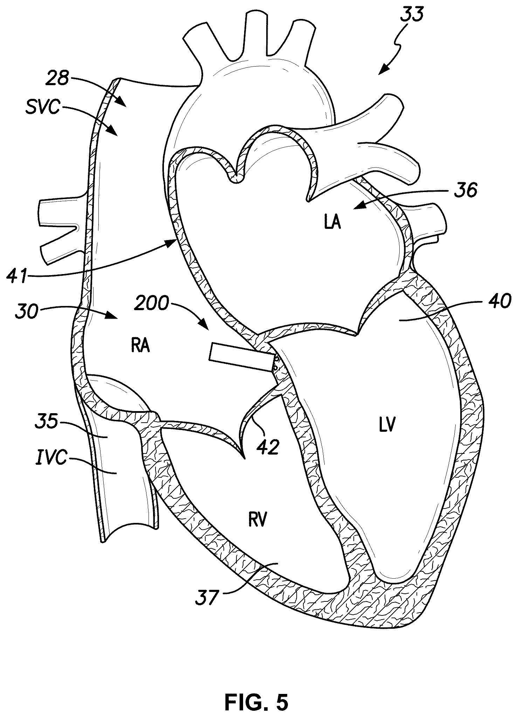

Figure 5 illustrates a sectional view of a patient's heart 33 with the leadless pacemaker 200 implanted therein according to an example application. The leadless pacemaker 200 has been placed through the superior vena cava 28 into the right atrium (RA) 30 of the heart 33. Figure 5 also shows the inferior vena cava 35, the left atrium 36, the right ventricle 37, the left ventricle 40, the atrial septum 41 that divides the two atria 30, 36, the ventricular vestibule VV, the right atrial appendage (RAA), and the tricuspid valve 42 between the right atrium 30 and the right ventricle 37. The view of Figure 5 is simplified and somewhat schematic, but is meant to illustrate an example implant placement. The term "septum" shall be used throughout to generally refer to any portion of the heart separating two chambers (e.g. RA to LA, RV to LV). The leadless pacemaker 200 is implanted entirely within a single local chamber of the heart. In Figure 5, the leadless pacemaker 200 may be implanted entirely and solely within the right atrium. In another example, the leadless pacemaker 200 is implanted entirely and solely within the right ventricle. In other examples, the leadless pacemaker 200 may be implanted entirely and solely within the left atrium or left ventricle via more invasive implant methods.

As used herein, the chamber in which the leadless pacemaker 200 is implanted shall be referred to as the "local" chamber. The local chamber includes a local chamber wall that is physiologically response to local activation events originating in the local chamber. The local chamber is at least partially surrounded by local wall tissue that forms or constitutes at least part of a conduction network for the associated chamber. For example, during normal operation, the wall tissue of the right atrium contracts in response to an intrinsic local activation event that originates at the sinoatrial (SA) node and in response to conduction that propagates along the atrial wall tissue. For example, tissue of the right atrium chamber wall in a healthy heart follows a conduction pattern, through depolarization, that originates at the SA node and moves downward about the right atrium until reaching the atria ventricular (AV) node. The conduction pattern moves along the chamber wall as the right atrium wall contracts.

In the example of Figure 5, the leadless pacemaker 200 is implanted in the right atrium. Optionally, the leadless pacemaker 200 may be mounted to the atrial tissue in an area near the triangle of Koch that is adjacent the ventricular vestibule. The conductive network of the tissue in the ventricular vestibule follows the conductive pattern of the right ventricle. In another example, the leadless pacemaker 200 may be positioned with the distal end fixated to the RA wall above the mitral valve. The leadless pacemaker 200 may be implanted within other chambers of the heart, outside of the RA, in other example use applications.

Figure 6 is a cross-sectional view of the leadless pacemaker 200 showing a first deployment mechanism 300 according to an embodiment. The deployment mechanism 300 in Figure 6 is an example deployment mechanism 108 shown in Figure 1. The deployment mechanism 300 is able to reversibly extend and retract the fixation tines 106. For example, the deployment mechanism 300 can extend the fixation tines 106 to achieve the deployed state from the non-deployed state, and the deployment mechanism 300 can retract the fixation tines 106 to achieve the non-deployed state from the deployed state. The deployment mechanism 300 can be controlled via operator input. The deployment mechanism 300 can also position the fixation tines 106 at multiple different deployed positions relative to the housing 102. For example, the deployment mechanism 300 can position the fixation tines 106 to project beyond the distal face 112 at one of multiple different incremental lengths or distances. As an example, the deployment mechanism 300 can control the fixation tines 106 to project from the distal face 112 a distance of 0.5 mm, 0.75 mm, 1 mm, 1.25 mm, and 1.5 mm. An operator may select the distance that the fixation tines 106 project from the housing 102 in the deployed state based on various factors, such as patient characteristics, characteristics of the target implant location (e.g., wall thickness), and/or the like.

The deployment mechanism 300 functions by converting rotational movement of a first component 302 to linear movement of a second component 304 that is coupled to the first component 302. The second component 304 is coupled to the fixation tines 106. In an example, the fixation tines 106 are mounted on a base 306, which may be a plate, disc, or the like. The base 306 is secured to the second component 304. In an example, the base 306 is bonded via an adhesive to the second component 304. The first component 302 is rotated by an external force. For example, the first component 302 may be rotated by a delivery assembly that is used to implant the leadless pacemaker 200. The delivery assembly may include a thin, rod-like tool, such as a stylet, a guide wire, a catheter, or the like that can engage and rotate the first component 302. The first component 302 is coupled to the second component 304 so that the rotation of the first component 302 causes the second component 304 to move linearly along a longitudinal or central axis of the leadless pacemaker 200. The linear motion of the second component 304 is relative to the first component 302. The linear motion of the second component 304 forces the base 306 and the fixation tines 106 to move linearly with the second component 304. The base 306 and the fixation tines 106 linearly translate relative to the distal tip 110 of the housing 102.

In an example, at least one of the first and second components 302, 304 is a threaded shaft. In Figure 6, the first component 302 is a threaded shaft, and the second component 304 is a nut that contains one or more ball bearings 308. The ball bearing(s) 308 engage a helical groove 310 of the threaded shaft. The ball bearing(s) 308 provide a mechanical interface between the threaded shaft and the nut with limited friction. Rotation of the first component 302 in a first direction (e.g., clockwise) forces the second component 304, the base 306, and the fixation tines 106 to move in a distal direction 312. Movement in the distal direction 312 extends the fixation tines 106, such as to transition from the non-deployed state to the deployed state. Rotation of the first component 302 in the opposite, second direction (e.g., counter-clockwise) forces the second component 304, the base 306, and the fixation tines 106 to move in a proximal direction 314. Movement in the proximal direction 314 retracts the fixation tines 106, such as to transition from the deployed state to the non-deployed state. An operator can make incremental changes to the projection distance of the fixation tines 106 by rotating the first component 302 to a minor extent.

In the illustrated example, the threaded shaft first component 302 forms a portion of the housing 102 along at least a proximal section of the leadless pacemaker 200. For example, the first component 302 includes the horn 204. Figure 7 is a distal view of the leadless pacemaker 200 of Figure 6 according to an embodiment. The horn 204 includes a drive feature 316. The drive feature 316 is a socket or depression that is sized and shaped to receive the distal tip of a tool that has a similar shape and a similar, though slightly smaller, size. The distal tip of a tool of the delivery assembly may be received within the drive feature 316 and rotated to exert rotational torque on the first component 302, causing the first component 302 to rotate. In the illustrated example, the drive feature 316 has a triangular shape, to accommodate a triangular distal tip of the tool. In other examples, the drive feature 316 may have different shapes, such as a square, a plus sign, a star, a hexagon, or the like.

In another embodiment, the first component 302 may be discrete and separate from the housing 102. For example, the housing 102 along the proximal section may be a hollow cylinder, and the first component 302 may be a threaded shaft that is contained within the hollow cylinder. The cylinder of the housing 102 may have a hole or aperture that permits a tool to enter the housing 102 to engage and rotate the first component 302.

Figure 8 illustrates a variation of the first deployment mechanism 300a in which the first component 302 is discrete from the housing 102. Like the first deployment mechanism 300 shown in Figure 6, the first component 302 is rotated by an applied force, which causes the second component 304 to linearly translate. The second component 304 may be the same as in Figure 6. The first component 302 is a threaded shaft that is located within the hollow cylindrical housing 102. The housing 102 defines an aperture 320 that permits a thin, rod-like tool 326, such as a stylet or guide wire, to enter the housing 102. The first component 302 is a threaded shaft. The helical threads of the first component 302 are not visible in Figure 8. In an example, the first component 302 includes a shoulder 322 that projects radially outward from an outer surface 324 of the first component 302. The shoulder 322 has an angled cam surface 328. The distal end 332 of the tool 326 abuts the angled cam surface 328. Force applied by the tool 326 in the distal direction 312 forces the distal end 332 to slide along the angled cam surface 328, which rotates the first component 302 in a first rotational direction 330 (e.g., counter-clockwise). In an example, rotation of the first component 302 in the first rotational direction 330 may cause the second component 304, the base 306, and the fixation tines 106 to move in the distal direction 312, transitioning from the non-deployed state to the deployed state.

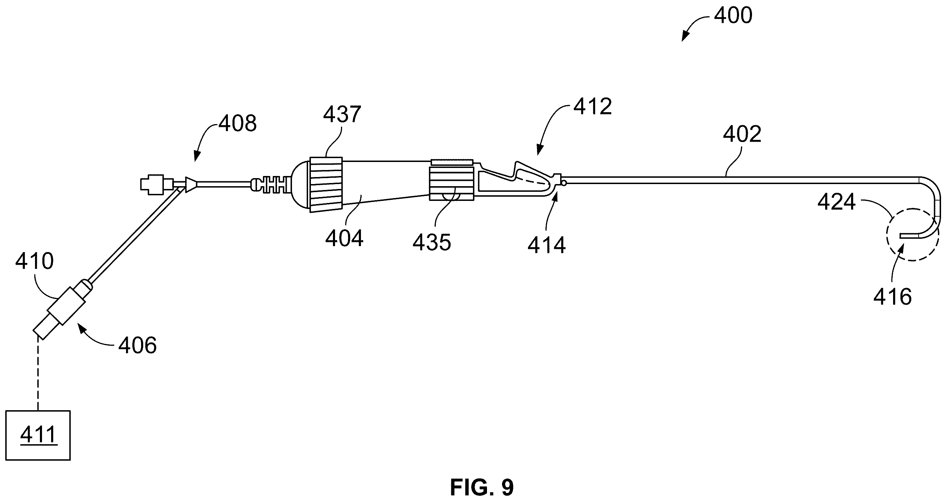

Figure 9 illustrates a delivery assembly 400 that can be used to implant the IMD 100 into a patient. The delivery assembly 400 may be formed according to an embodiment to control the deployment mechanism 300 and/or 300a (shown in Figures 6 and 8). For example, the delivery assembly 400 can be actuated by an operator to rotate the first component 302, thereby extending (or retracting) the fixation tines 106. The delivery assembly 400 in the illustrated example includes a catheter (or introducer sheath) 402, a handle 404, a connector assembly 406, and a fluid flushing assembly 408. The delivery assembly 400 includes additional components not visible in Figure 9, such as the thin, rod-like tool 326 (shown in Figure 8). The delivery assembly 400 may have at least some different components in other examples. The fluid flushing assembly 408 may be omitted in at least one example. The delivery assembly 400 may also include an obturator/dilator.

The connector assembly 406 may include an electrical connector 410. In an example, the electrical connector 410 may be electrically connected to one or more electrodes of the IMD 100. The connector assembly 106 may communicatively connect to a pacing system analyzer device 411. The pacing system analyzer device 411 may use the IMD 100 to electrically map multiple candidate sites of interest (SOI) along patient cardiac tissue to select a target implant location for long-term implant of the IMD 100. For example, the pacing system analyzer device 411 may include a pulse generator for generating pacing pulses, a memory device, and processing circuitry for analyzing signals indicative of an evoked response (to the pacing pulses) in the myocardial tissue.

The handle 404 may include a hemostasis hub 412 for accepting and coupling to (e.g., tethering to) a proximal end 414 of the catheter 402. The catheter 402 extends from the proximal end 414 to a distal end 416 of the catheter 402. In an embodiment, the catheter 402 has at least one lumen that extends through the catheter 402 from the proximal end 414 to the distal end 416 and is open at both ends 414, 416. The hemostasis hub 412 permits access to the lumen of the catheter 402. The fluid flushing assembly 408 may mechanically couple to the hemostasis hub 412 and fluidly couple to the lumen through the hemostasis hub 412.

The catheter 402 is configured to introduce the IMD 100 into a designated anatomical region of a patient, such as the heart. The catheter 402 may be steerable to navigate through a tortuous vascular system of the patient. The catheter 402 may be steered to position a distal end segment 424 of the delivery assembly 400 proximate to a SOI, with the distal end of the catheter 402 facing the cardiac tissue at the SOI. The catheter 402 may include a plurality of sheath segments or portions along its length, and at least some of the sheath segments may be bendable relative to other sheath segments. An operator may steer the catheter 402 by holding the handle 404 and manipulating a first user input device 435 coupled to the handle 404.

In an example, the IMD 100 may be held by the distal end segment 424 of the catheter 402 during an implant procedure while the distal end segment 424 is proximate to the SOI. The thin, rod-like tool 326 may be advanced through the lumen along the length of the catheter 402 until the distal end 332 of the tool 326 engages the angled cam surface 328 as shown in Figure 8 or the drive feature 316 shown in Figures 6 and 7. The thin, rod-like tool 326 may be mechanically connected to a second user input device 437. For example, a proximal end of the tool 326 may be mechanically connected to the second user input device 437, while the distal end 332 engages the angled cam surface 328 or the drive feature 316. In an example, the second user input device 437 may be coupled to the handle 404. The second user input device 437 may be a rotatable dial, a lever, a switch, or the like. In the example shown in Figures 6 and 7, in which rotation of the tool 326 rotates the first component 302, the second user input device 437 may be a rotatable dial. Rotation of the dial may exert rotational torque on the tool 326. In the example shown in Figure 8, in which distal translation of the tool 326 forces the first component 302 to rotate, the second user input device 437 may be a lever. For example, pressing or squeezing the lever may push the tool 326 in the distal direction 312, which forces the first component 302 to rotate. As a result, once the distal end of the tool 326 is coupled to the IMD 100 (e.g., the leadless pacemaker 200), an operator holding the handle 404 of the delivery assembly 400 may selectively control the deployment mechanism 300, 300a to deploy the fixation tines 106 by manipulating the second user input device 437.

Both deployment mechanisms 300, 300a may be able to extend the fixation tines 106 to different protruding distances relative to the distal face 112 of the distal tip 110 in the deployed state, based on varying operator input on the second user input device 437. For example, greater rotation of the dial in a first rotational direction may cause the fixation tines 106 to extend to a first protruding distance that is greater than a second protruding distance caused by less rotation of the dial. In another example, pushing the lever different distances may affect the amount of linear movement of the tool 326. As such, the first component 302 may be rotated to different extents based on the force exerted on and/or the travel of the lever.

In another example, the second user input device 437 may be a button or key that is electrically connected to a solenoid in the delivery assembly 400. For example, the solenoid may be housed within the handle 404 or coupled to the handle 404. The solenoid may be coupled to the proximal end of the thin, rod-like tool 326. In an example, physical actuation of the button or key may cause the solenoid to either receive power (e.g., electrical energy) or lose power, which forces the tool 326 in the distal direction. In this example, electrical energy provides the drive force on the tool 326, which causes the deployment of the fixation tines 106 via the deployment mechanism 300, 300a.

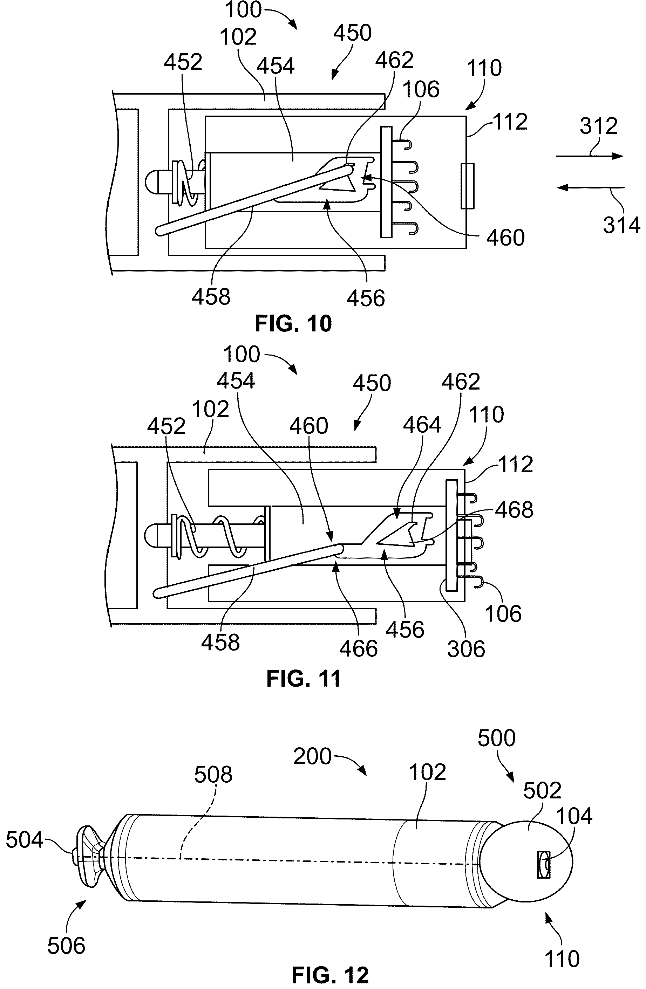

Figure 10 illustrates a cross-sectional view of a distal portion of the IMD 100 with the fixation tines 106 in a non-deployed state according to another embodiment. Figure 11 illustrates a cross-sectional view of the IMD 100 in Figure 10 with the fixation tines 106 in the deployed state. The IMD 100 in Figures 10 and 11 has a second type of deployment mechanism 450. The deployment mechanism 450 in Figure 10 is an example deployment mechanism 108 shown in Figure 1. The deployment mechanism 450 is able to reversibly extend and retract the fixation tines 106 to achieve the deployed state and the non-deployed state, respectively. The deployment mechanism 450 can be controlled via operator input. Unlike the deployment mechanisms 300, 300a, the deployment mechanism 450 does not rely on physical force provided by a rod-like tool of the delivery assembly. The deployment mechanism 450 is located at or proximate to the distal tip 110 of the housing 102.

The deployment mechanism 450 is (or includes) a biased, push-actuated release system. The push-actuated release system (referred to herein as "release system") may function similar to a retractable ballpoint pen that operates by a user pushing an element of the pen. Release system 450 may be contained within the housing 102. The release system 450 may force the fixation tines 106 to travel in the distal direction 312 from the non-deployed state (Figure 10) to the deployed state (Figure 11) in response to a first application of force exerted on the distal tip 110. Figure 10 shows the release system 450 in a held state. The first application of force may be at least partially in the proximal direction 314. The first force application causes the distal tip 110 of the housing 102 to retract in the proximal direction 314 for at least a threshold distance. By surpassing the threshold distance, the release system 450 achieves a released state. Once released, a biasing member 452 of the release system 450 can move the fixation tines 106, via the base 306, in the distal direction 312 so that the fixation tines 106 project beyond the distal face 112 of the distal tip 110. The biasing member 452 may be a spring or another elastic material that exerts a resilient, biasing force on the base 306. In another example, the biasing member 452 may be a shape memory material that forces the base 306 to move in the distal direction 312 when the shape memory material is exposed to a particular stimulus. The stimulus may be a temperature that is above a threshold temperature, a temperature that is below a threshold temperature, or the like.

While the fixation tines 106 are in the deployed state, as shown in Figure 11, the release system 450 may force the fixation tines 106 to retract in the proximal direction 314 from the deployed state to the non-deployed state in response to a second application of force exerted on the distal tip 110. The second force application causes the distal tip 110 to retract in the proximal direction 314 for at least the threshold distance. As a result, the fixation tines 106 can be repeatedly alternated between the deployed state and the non-deployed state by sequentially depressing the distal tip 110 in the proximal direction 314. The release system 450 may be referred to as a push-push mechanism because a first push deploys the fixation tines 106 and a second push in the same direction retracts the fixation tines 106. In use, the force that is applied to the distal tip 110 to initiate the transition may be accomplished by pushing the IMD 100 against a tissue wall of the patient. For example, the delivery system 400 may guide the IMD 100 to the tissue at a target implant location. The operator may control the delivery system 400 to push the distal tip 110 of the IMD 100 against the tissue wall so that the distal tip 110 receives an opposite force by the tissue wall in the proximal direction 314. An example tissue wall may be part of the right atrium. The delivery system 400 may push the IMD 110 against the tissue wall with sufficient force to cause the distal tip 110 of the housing 102 to retract for at least a threshold distance.

Figures 10 and 11 show one example of the release system deployment mechanism 450. The spring 452 exerts a constant biasing force on a can 454 of the deployment mechanism 450. The biasing force exerted by the spring 452 on the can 454 is in the distal direction 312. The base 306 and fixation tines 106 are mounted to the can 454, such as at a distal end of the can 454. The can 454 defines a track 456. The release system 450 includes a clip 458 that is pivotably mounted to the housing 102 at one end of the clip 458 and is confined within the track 456 at the other end of the clip 458. For example, a distal end 460 of the clip 458 may have, or be coupled to, a pin that extends into the track 456 and moves along the track 456.

When in the held state shown in Figure 10, the distal end 460 of the clip 458 abuts a catch tab 462, which holds the distal end 460 of the clip 458 in place. The clip 458 holds the can 454 at the position shown in Figure 10, even against the biasing force of the spring 452. By fixing the can 454 in place at the position shown in Figure 10, the fixation tines 106 are retained in the retracted, non-deployed state. A force application on the distal tip 110 in the proximal direction 314 may cause the distal tip 110 to move in the proximal direction 314, against the force of the spring 452. If the force is sufficient, the distal end 460 of the clip 458 releases from the catch tab 462 and moves into a release chute 464 of the track 456, as shown in Figure 11. For example, an angled surface of the track 456 may cause the distal end 460 of the clip 458 to slide into the release chute 464. When the applied force is relieved, the biasing force exerted by the spring 452 causes the can 454 to move in the distal direction 312. The distal movement of the can 454 extends the base 306 and fixation tines 106 to the deployed state, as shown in Figure 11. The distal end 460 of the clip 458 slides to a proximal end 466 of the track 456.

When a second applied force is received on the distal tip 110 of the housing 102, while the fixation tines 106 are in the deployed state, the fixation tines 106 will achieve the non-deployed state if the force is sufficient to move the can 454 in the proximal direction 314 at least a threshold distance. In Figure 11, the threshold distance is the distance that the distal end 460 travels from the proximal end 466 of the track 456 to a ledge 468 that extends to the catch tab 462. The length of the threshold distance may be exaggerated in Figures 10 and 11 for ease of description and understanding. Example threshold distances, which may represent the travel distance of the fixation tines 106 between the deployed and non-deployed states, may be 1 mm, 2 mm, 3 mm, 5 mm, 8 mm, 10 mm, or the like, including values between the stated values.

Figure 12 illustrates the leadless pacemaker 200 with the fixation tines 106 in a non-deployed state according to another embodiment. The leadless pacemaker 200 in Figure 12 has a third type of deployment mechanism 500. The deployment mechanism 500 in Figure 12 is an example deployment mechanism 108 shown in Figure 1. The leadless pacemaker 200 is an example IMD 100 shown in Figure 1. The deployment mechanism 500 includes a dissolvable cap 502 mounted to the distal tip 110. The dissolvable cap 502 covers the fixation tines 106 in the non-deployed state. The fixation tines 106 are not visible in Figure 12 because the fixation tines 106 are concealed by the dissolvable cap 502. In an example, the tip electrode 104 is exposed through an opening 504 in the dissolvable cap 502. The tip electrode 104 may protrude from the dissolvable cap 502 to permit the tip electrode 104 to be used for mapping candidate target implant locations prior deploying the fixation tines 106 for more permanently fixating the leadless pacemaker 200 to the patient tissue.

In an example, the deployment mechanism 500 does not extend or retract the fixation tines 106 relative to the housing 102. The fixation tines 106 are fixed relative to the housing 102. The fixation tines 106 project beyond the distal face 112, as shown for example in Figure 2. In the non-deployed state, the fixation tines 106 are embedded within and covered by the dissolvable cap 502, so the fixation tines 106 cannot penetrate and/or snag on patient tissue. In the deployed state, the dissolvable cap 502 dissolves to reveal and expose the fixation tines 106. The dissolvable cap 502 is composed of a biodegradable material. The biodegradable material may be selected to dissolve and expose the fixation tines 106 after a designated period of time in contact with biological fluid within the patient. The biodegradable material may be polymeric. Example biodegradable materials for the dissolvable cap 502 may include: (i) Polylactic acid (PLA) (ii) Polyglycolic acid (PGA), (iii) Poly(lactic-co-glycolic acid)(PLGA), (iv) Polycaprolactone (PCL), (v) Poly(trimethylene carbonate) (PTMC), and/or (vi) peptide-based hydrogels, including combinations (e.g., copolymers) thereof.

As an example, the dissolvable cap 502 may be composed of a biodegradable material that exposes the fixation tines 106 after approximately 20 minutes of exposure to the biological fluids (e.g., blood) of the patient. The operator has approximately 20 minutes then to navigate the leadless pacemaker 200 through the circulatory system and determine the target implant location, such as by mapping multiple different candidate sites. As the dissolvable cap 502 dissolves, the biodegradable material may be absorbed by the body. Once the fixation tines 106 are sufficient exposed (e.g., deployed), the operator may control the delivery assembly 400 to press the distal tip 110 against the patient tissue at the target implant location for the fixation tines 106 to bite into and fixate the pacemaker 200 to the patient tissue.

In the example described above, the dissolvable cap 502 may dissolve upon exposure to the biological fluids of the patient without any external catalyst. Optionally, the degradation of the dissolvable cap 502 may be accelerated or initiated by a catalyst. As one example, the catalyst may be an electric current that is conveyed to the dissolvable cap 502. The electric current may catalyze the degradation of the biodegradable material. For example, the deployment mechanism 500 may include an electrical contact 504 at a proximal tip 506 of the housing 102. The electrical contact 504 may be electrically connected to a wire 508 that extends from the electrical contact 504 to the dissolvable cap 502. The electrical contact 504 may electrically connect to an electrical element of the delivery assembly 400. The wire 508 may be any electrically conductive element. The wire 508 may be contained within the housing 102, so the wire 508 is shown in phantom in Figure 12. The electrical element of the delivery assembly 400 may be an electrical contact at the end of a thin, rod-like tool that is inserted through the catheter 402, or the like. Upon the operator providing a user input, the delivery assembly 400 may convey an electric current to the electrical contact 504, which is conveyed via the wire 508 to the dissolvable cap 502. The electric current supplied to the dissolvable cap 502 may catalyze the degradation process, thereby causing the deployed state of the fixation tines 106.

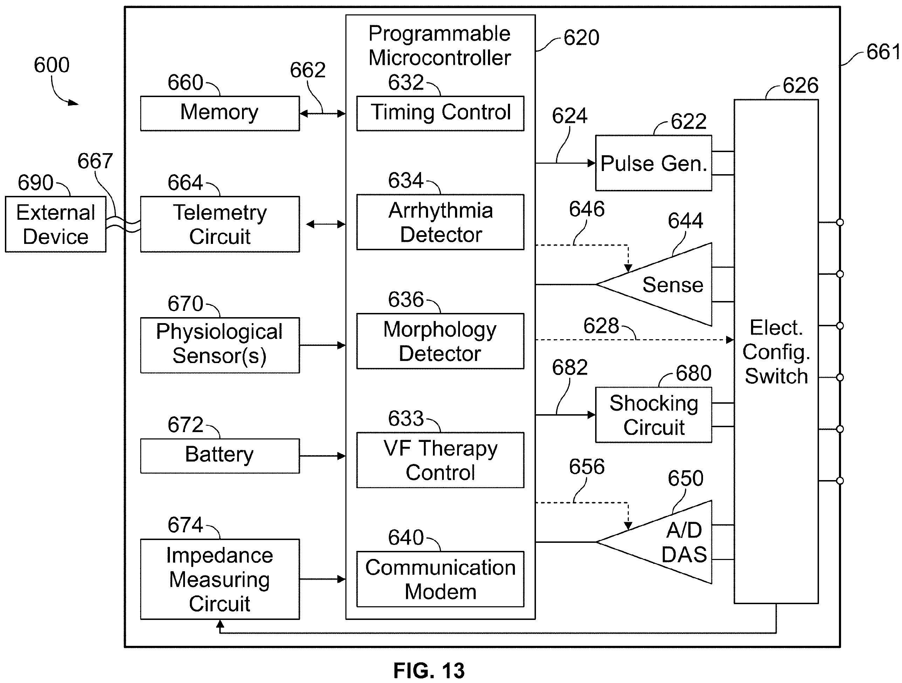

Figure 13 illustrates a block diagram of an exemplary IMD 600 that is configured to be implanted into the patient in accordance with embodiments herein. The IMD 600 may be an example IMD 100 shown in Figure 1. The IMD 600 may be the leadless pacemaker 200 shown in any of Figures 4, 5, 6, 7, 8, or 12). For example, the components shown and described with reference to Figure 13 may be internal components of the leadless pacemaker 200 within the housing 102, other than the deployment mechanism 108 and the fixation tines 106, which are already described herein. The IMD 600 may treat both fast and slow arrhythmias with stimulation therapy, including cardioversion, pacing stimulation, an implantable cardioverter defibrillator, suspend tachycardia detection, tachyarrhythmia therapy, and/or the like.

The IMD 600 has a housing 661 to hold the electronic/computing components. The housing 661 may be the housing 102 shown in Figure 1, and more specifically may be the housing 102 of the leadless pacemaker 200. The housing 661 (which is often referred to as the "can," "case," "encasing," or "case electrode") may be programmably selected to act as the return electrode for certain stimulus modes. The housing 661 may hold and/or connect to one or more electrodes, such as the tip electrode 104. The type and location of each electrode may vary. For example, the electrodes may include various combinations of ring, tip, coil, shocking electrodes, and the like.

The IMD 600 includes a programmable microcontroller 620 that controls various operations of the IMD 600, including cardiac monitoring and stimulation therapy. The microcontroller 620 includes a microprocessor (or equivalent control circuitry), one or more processors, RAM and/or ROM memory, logic and timing circuitry, state machine circuitry, and I/O circuitry. The IMD 600 further includes a pulse generator 622 that generates electrical stimulation in the form of pulses. The IMD 600 includes an electrode configuration switch 626 for connecting the desired electrodes to the appropriate I/O circuits, thereby facilitating electrode programmability. The switch 626 is controlled by a control signal 628 from the microcontroller 620. In an example, the pulse generator 622 is controlled to generate electrical stimulation that is delivered, via the tip electrode 104, to the tissue of the patient when the IMD 600 is secured to the patient tissue via the fixation tines 106.

The IMD 600 includes at least one sensing circuit 644 selectively coupled to one or more electrodes that perform sensing operations, through the switch 626, to detect the presence of cardiac activity signals in the chamber of the heart. The output of the sensing circuit 644 is connected to the microcontroller 620. The microcontroller 620 may trigger or inhibit the pulse generator 622 in response to the absence or presence of cardiac activity signals detected by the sensing circuit 644. The sensing circuit 644 may receive a control signal 646 from the microcontroller 620 for purposes of controlling the gain, threshold, polarization charge removal circuitry (not shown), and the timing of any blocking circuitry (not shown) coupled to the inputs of the sensing circuit 644. The sensing circuit 644 may operate in, for example, a unipolar sensing configuration or a bipolar sensing configuration.

The IMD 600 may include an analog-to-digital (A/D) data acquisition system (DAS) 650 coupled to one or more electrodes via the switch 626 to sample cardiac signals across any pair of desired electrodes. The A/D converter 650 may acquire intracardiac electrogram signals, convert the raw analog data into digital data, and then store the digital data. The digital data may be stored for later processing and/or telemetric transmission to an external device 690 (e.g., a programmer, local transceiver, or a diagnostic system analyzer). The A/D converter 650 is controlled by a control signal 656 from the microcontroller 620.

The microcontroller 620 is operably coupled to a memory 660 by a suitable data/address bus 662. The programmable operating parameters used by the microcontroller 620 are stored in the memory 660 and used to customize the operation of the IMD 600 to suit the needs of a particular patient. The operating parameters of the IMD 600 may be non-invasively programmed into the memory 660 through a telemetry circuit 664 in telemetric communication via communication link 667 (e.g., MICS, Bluetooth low energy, and/or the like) with the external device 690.

The IMD 600 can further include one or more physiological sensors 670. Such sensors are commonly referred to as "rate-responsive" sensors because they are typically used to adjust pacing stimulation rates according to the exercise state of the patient. However, the physiological sensor 670 may further be used to detect changes in cardiac output, changes in the physiological condition of the heart, or diurnal changes in activity (e.g., detecting sleep and wake states). Signals generated by the physiological sensors 670 are passed to the microcontroller 620 for analysis. While shown as being included within the IMD 600, the physiological sensor(s) 670 may be external to the IMD 600, yet still, be implanted within or carried by the patient. Examples of physiological sensors might include sensors that, for example, sense respiration rate, pH of blood, ventricular gradient, activity, position/posture, minute ventilation, and/or the like.

A battery 672 may provide operating power to all of the components in the IMD 600. The battery 672 is capable of operating at low current drains for long periods of time, and is capable of providing a high-current pulses (for capacitor charging) when the patient requires a shock pulse (e.g., in excess of 2 A, at voltages above 2 V, for periods of 10 seconds or more). The battery 672 also desirably has a predictable discharge characteristic so that elective replacement time can be detected. As one example, the IMD 600 employs lithium/silver vanadium oxide batteries.

The IMD 600 further includes an impedance measuring circuit 674, which can be used for many things, including sensing respiration phase. The impedance measuring circuit 674 is coupled to the switch 626 so that any desired electrode and/or terminal may be used to measure impedance in connection with monitoring respiration phase. The IMD 600 may be equipped with a communication modem (modulator/demodulator) 640 to enable wireless communication with other devices, implanted devices and/or external devices. In one implementation, the communication modem 640 may use high frequency modulation of a signal transmitted between a pair of electrodes. As one example, the signals may be transmitted in a high frequency range of approximately 10-80 kHz, as such signals travel through the body tissue and fluids without stimulating the heart or being felt by the patient.

Optionally, the microcontroller 620 may control a shocking/therapy circuit 680 by way of a timing control 632. The shocking/therapy circuit 680 generates shocking pulses as controlled by the microcontroller 620. The shocking circuit 680 may be controlled by the microcontroller 620 generating a control signal 682.

Although not shown, the microcontroller 620 may further include other dedicated circuitry and/or firmware/software components that assist in monitoring various conditions of the patient's heart and managing pacing therapies. The microcontroller 620 may include a timing control 632, an arrhythmia detector 634, a morphology detector 636, and a ventricular fibrillation (VF) therapy controller 633. The timing control 632 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The morphology detector 636 is configured to review and analyze one or more features of the morphology of cardiac activity signals. For example, the morphology detector 636 may analyze the morphology of detected R waves to identify non- conducted ventricular events, such as ventricular fibrillation and the like. The arrhythmia detector 634 may be configured to apply one or more arrhythmia detection algorithms for detecting arrhythmia conditions. By way of example, the arrhythmia detector 634 may apply various detection algorithms. The arrhythmia detector 634 may be configured to declare a ventricular fibrillation episode based on the cardiac events. The therapy controller 633 may identify a multi-phase therapy based on the ventricular fibrillation episode. The therapy controller 633 may manage delivery of burst pacing therapy at the implant location in a coordinated manner after one or more shocks.

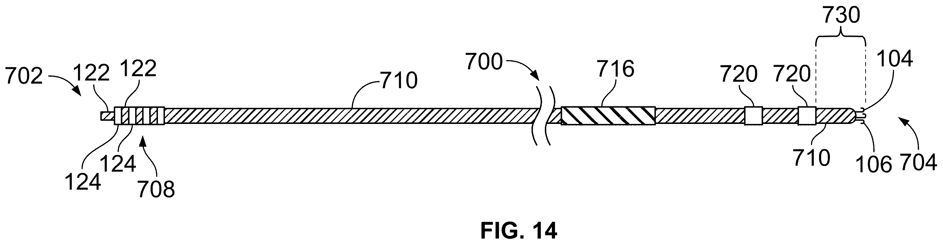

Although various embodiments described herein refer to the IMD 100 as a leadless pacemaker, the IMD 100 is not limited to leadless pacemakers. For example, the IMD 100 may be a transvenous lead. The distal portion of the IMD 100 shown in Figure 2 may represent the distal end segment of the transvenous lead. Figure 14 illustrates an implantable lead 700 according to an embodiment. The implantable lead 700 may be a transvenous lead that is configured to be implanted within the heart, such as within the right atrium. The implantable lead 700 represents an example IMD 100 shown in Figure 1. For example, the fixation tines 106 and deployment mechanism 108 may be located at a distal segment 730 of the lead 700. The implantable lead 700 extends from a proximal end 702 to a distal end 704. The fixation tines 106 are located at the distal end 704 for fixating the distal end 704 to patient tissue at a target implant location. The deployment mechanism 108 is not visible in Figure 14. The deployment mechanism 108 of the lead 700 may be any of the deployment mechanisms shown and described herein.

The implantable lead 700 may include a terminal connection segment 708 at the proximal end 702. The terminal connection segment 708 is designed to be received in a receptacle or lead adaptor of a pulse generator device that is implantable within the patient at a location remote from the heart. The terminal connection segment 708 may include electrically conductive contacts 722 and electrically insulating portions 724 that alternate with the electrically conductive contacts 722 in a row. A lead body 710 extends between the proximal end 702 and the distal end 704, such as between the terminal connection segment 708 and the fixation tines 106. The implantable lead 700 may include multiple electrodes for delivering electrical stimulation to the cardiac tissue of the patient. For example, the implantable lead 700 may include a defibrillation (e.g., shock) coil 716, the tip electrode 104, and one or more ring electrodes 720. The electrodes may be spaced apart along the length of the implantable lead 700. The ring electrode(s) 720 may be disposed between the defibrillation coil 716 and the tip electrode 104. The implantable lead 700 includes electrical conductors that extend through the lead body 710 from the electrodes 716, 104, 720 to corresponding electrically conductive contacts 722 of the terminal connection segment 708. The electrical conductors may be helical coils, multi-filar conductors, and/or the like. Pacing pulses from the pulse generator device may be conveyed through one or more of the electrical conductors to at least one of the ring and tip electrodes 718, 104 for delivery to the cardiac tissue. Defibrillation shocks from the pulse generator device may be conveyed through one or more of the electrical conductors to the shock/defibrillation coil 716 to shock the cardiac tissue.

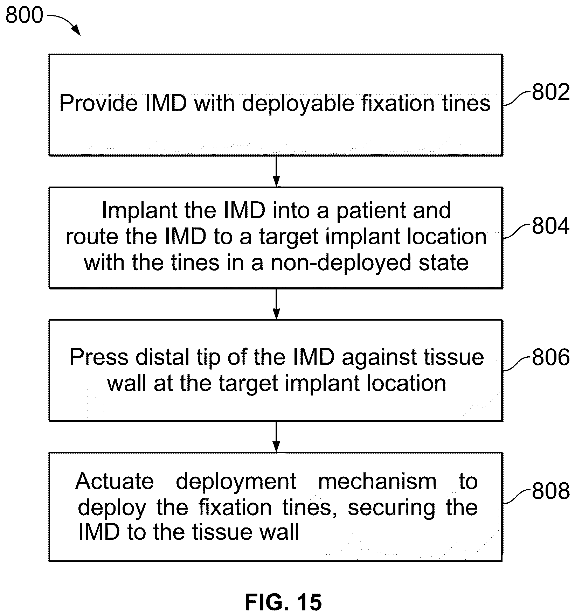

Figure 15 is a flowchart of a method 800 of fixating an IMD to patient tissue according to an embodiment. The method 800 may use the IMD 100 shown in Figure 1, and any of the versions of the IMD 100 shown in Figures 2 through 14. In different embodiments, the method may include different steps not shown in Figure 15, may omit one or more of the steps shown in Figure 15, and/or may have a different order of the steps than shown in Figure 15.

At step 802, an IMD 100 is provided that includes deployable fixation tines 106. The fixation tines 106 are mounted to a distal tip 110 of a housing 102 of the IMD 100. The fixation tines 106 have hook features 120 for securing to tissue of the patient. The IMD 100 also has a deployment mechanism 108 configured to selectively deploy the fixation tines 106 from a non-deployed state to a deployed state. In the deployed state, the fixation tines 106 are exposed along a distal face 112 of the housing 102. In the non-deployed state, the fixation tines 106 are concealed and not exposed along the distal face 112. The deployment mechanism 108 is an integral component of the IMD 100. For example, the deployment mechanism 108 may be part of the housing 102 and/or contained within the housing 102. The deployment mechanism 108 is not a discrete sheath or sleeve that can be retracted relative to the IMD 100 to expose the fixation tines 106.

At step 804, the IMD 100 is implanted into a patient. The IMD 100 may be implanted using a delivery assembly 400. The delivery assembly 400 may route the IMD 100 through the circulatory system of the patient to a target implant location. The target implant location, in an example, may be the right atrium. The fixation tines 106 are in the non-deployed state during the implant process as the IMD 100 is routed to the target implant location.

At step 806, the IMD 100 is pressed into a tissue wall of the patient at the target implant location. At step 808, the deployment mechanism 108 is actuated to deploy the fixation tines 106 once the IMD 100 is at the target implant location. Upon deploying, the fixation tines 106 penetrate and embed in the tissue, securing the IMD 100 to the tissue wall.

In an example, the deployment mechanism 108 may be actuatable a second time, while the IMD 100 is mounted to the tissue wall, to retract the fixation tines 106 to the non-deployed state. The retraction of the fixation tines 106 enable the IMD 100 to be extricated from the tissue for retrieval of the IMD 100 out of the patient or repositioning of the IMD 100 within the patient.

A method is disclosed for fixating an implantable medical device at a target implant location within a patient. The method comprises providing an implantable medical device (IMD) having a housing including a distal tip that has a distal face, the IMD having a plurality of fixation tines mounted to the distal tip of the housing and comprising hook features for securing the IMD to tissue of the patient, the IMD having a deployment mechanism configured to selectively move the fixation tines between a deployed state and a non-deployed state; utilizing a delivery assembly to route the IMD to a target implant location within the patient, where the fixation tines are in the non-deployed state during an implant process as the IMD is routed to the target implant location; pressed the distal tip of the IMD against a tissue wall of the patient at the target implant location; and actuating the deployment mechanism to deploy the fixation tines once the IMD is at the target implant location, wherein upon deploying, the fixation tines penetrate and embed in the tissue securing the IMD at a target implant location.

Additionally, or alternatively, the method further comprises providing a tip electrode mounted to the distal tip of the housing and exposed along the distal face, the tip electrode configured to contact the tissue in connection with at least one of sensing cardiac activity signals or delivering therapy.

Additionally, or alternatively, the method further comprises actuating the deployment mechanism to selectively move the fixation tines in a distal direction relative to the distal tip of the housing from a non-deployed state to the deployed state, wherein the fixation tines in the deployed state extend beyond the distal face so that the hook features are exposed for securing to the tissue of the patient, and the hook features of the fixation tines in the non-deployed state are not exposed for securing to the tissue of the patient.

Additionally, or alternatively, the method further comprises actuating the deployment mechanism to selectively retract the fixation tines relative to the distal tip of the housing from the deployed state to the non-deployed state.

Additionally, or alternatively, the method further comprises mechanically connecting the delivery mechanism to a user input device on a delivery assembly used to implant the IMD, actuating the deployment mechanism to extend the fixation tines to different protruding distances relative to the distal face of the distal tip in the deployed state, based on varying operator input on the user input device.

Additionally, or alternatively, the method further comprises providing the deployment mechanism to include a first component and a second component that is coupled to the first component, the second component coupled to a base that holds the fixation tines, the method further comprising rotating the first component by the delivery assembly, wherein rotation of the first component causes the second component to linearly translate relative to the first component, forcing the base and the fixation tines to linearly translate relative to the distal tip of the housing.

Additionally, or alternatively, one of the first component or the second component is a threaded shaft, and the other of the first component or the second component is a nut that contains one or more ball bearings that engage a helical groove of the threaded shaft.

Additionally, or alternatively, the first component that is configured to be rotated by the delivery assembly includes an angled cam surface, the angled cam surface configured to be contacted by a distal end of a rod-like tool of the delivery assembly, wherein the first component is configured to rotate due to translation of the rod-like tool in the distal direction.

Additionally, or alternatively, the deployment mechanism is a push-actuated release system within the housing of the IMD, wherein the push-actuated release system is configured to force the fixation tines to travel in the distal direction from the non-deployed state to the deployed state in response to a first application of force exerted on the distal tip that causes the distal tip to retract in a proximal direction for at least a threshold distance.

Additionally, or alternatively, while the fixation tines are in the deployed state, the push-actuated release system is configured to force the fixation tines to retract in the proximal direction from the deployed state to the non-deployed state in response to a second application of force exerted on the distal tip that causes the distal tip to retract in the proximal direction for at least the threshold distance.

Additionally, or alternatively, the deployment mechanism includes a dissolvable cap mounted to the distal tip, the dissolvable cap covering the fixation tines in a non-deployed state, the dissolvable cap composed of a biodegradable material configured to dissolve and expose the fixation tines, achieving the deployed state, after a designated period of time in contact with biological fluid within the patient.

Additionally, or alternatively, the deployment mechanism includes a dissolvable cap mounted to the distal tip, an electrical contact at a proximal end of the housing, and a wire that extends from the electrical contact to the dissolvable cap, the dissolvable cap covering the fixation tines in a non-deployed state of the fixation tines, wherein the electrical contact is configured to electrically connect to an electrical element of the delivery assembly and receive an electric current from the electrical element that is conveyed via the wire to the dissolvable cap, the dissolvable cap comprising a polymer material configured to dissolve and expose the fixation tines, achieving the deployed state of the fixation tines, in response to receiving the electric current.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An implantable medical device, IMD, (100) configured to be implanted within a patient, the IMD (100) comprising:
a housing (102) including a distal tip (110) that has a distal face (112);
a tip electrode (104) mounted to the distal tip (110) of the housing (102) and exposed along the distal face (112);
a plurality of fixation tines (106) mounted to the distal tip (110) of the housing (102) and comprising hook features (120) for securing to tissue of the patient; and
a deployment mechanism (108, 300, 300a, 450, 500) configured to selectively deploy the fixation tines (106) to a deployed state, wherein the fixation tines (106) in the deployed state are exposed along the distal face (112) and configured to embed into the tissue of the patient to secure the IMD (100) at a target implant location.

2. The IMD of claim 1, wherein the tip electrode (104) is centered along the distal face (112) of the distal tip (110), and the fixation tines (106) surround the tip electrode (104).

3. The IMD of claim 2, wherein
the fixation tines (106) are annularly arranged in a ring; or
at least some of the fixation tines (106) are located at different radial distances from the tip electrode (104).

4. The IMD of any one of claims 1 to 3, wherein the fixation tines (106) in the deployed state do not outwardly extend beyond a diameter of the housing (102).

5. The IMD of any one of claims 1 to 4, wherein the fixation tines (106) are J-shaped.

6. The IMD of any one of claims 1 to 5, wherein the plurality of fixation tines (106) includes at least ten fixation tines.

7. The IMD of any one of claims 1 to 6, wherein the IMD (100) is a leadless pacemaker (200) comprising a pulse generator (622) configured to generate electrical stimulation that is delivered, via the tip electrode (104), to the tissue of the patient.

8. The IMD of any one of claims 1 to 6, wherein the IMD (100) is a transvenous lead (700).

9. The IMD of any one of claims 1 to 8, wherein the deployment mechanism (108, 300, 300a, 450, 500) is configured to selectively move the fixation tines (106) in a distal direction relative to the distal tip (110) of the housing (102) from a non-deployed state to the deployed state, wherein the fixation tines (106) in the deployed state extend beyond the distal face (112) so that the hook features (120) are exposed for securing to the tissue of the patient, and the hook features (120) of the fixation tines (106) in the non-deployed state are not exposed for securing to the tissue of the patient.

10. The IMD of any one of claims 1 to 9, wherein the deployment mechanism (108, 300, 300a) is configured to mechanically connect to a user input device (435) on a delivery assembly (400) used to implant the IMD (100), and the deployment mechanism (108, 300, 300a) is configured to extend the fixation tines (106) to different protruding distances relative to the distal face (112) of the distal tip (110) in the deployed state, based on varying operator input on the user input device (435).

11. The IMD of claim 10, wherein the deployment mechanism (108, 300, 300a) includes a first component (302) and a second component (304) that is coupled to the first component (302), the second component (304) coupled to a base (306) that holds the fixation tines (106), wherein the first component (302) is configured to be rotated by the delivery assembly (400), and rotation of the first component (302) causes the second component (304) to linearly translate relative to the first component (302), forcing the base and the fixation tines (106) to linearly translate relative to the distal tip (110) of the housing (102).

12. The IMD of claim 11, wherein
one of the first component (302) or the second component (304) is a threaded shaft, and the other of the first component (302) or the second component (304) is a nut that contains one or more ball bearings (308) that engage a helical groove of the threaded shaft; and/or
the first component (302) that is configured to be rotated by the delivery assembly (400) includes an angled cam surface (328), the angled cam surface (328) configured to be contacted by a distal end (332) of a rod-like tool (326) of the delivery assembly (400), wherein the first component (302) is configured to rotate due to translation of the rod-like tool (326) in the distal direction.

13. The IMD of any one of claims 1 to 9, wherein the deployment mechanism (108, 450) is a push-actuated release system (450) within the housing (102) of the IMD (100), wherein the push-actuated release system (450) is configured to force the fixation tines (106) to travel in the distal direction from the non-deployed state to the deployed state in response to a first application of force exerted on the distal tip (110) that causes the distal tip (110) to retract in a proximal direction for at least a threshold distance, preferably wherein, while the fixation tines (106) are in the deployed state, the push-actuated release system (450) is configured to force the fixation tines (106) to retract in the proximal direction from the deployed state to the non-deployed state in response to a second application of force exerted on the distal tip (110) that causes the distal tip (110) to retract in the proximal direction for at least the threshold distance.

14. The IMD of any one of claims 1 to 9, wherein the deployment mechanism (108, 500) includes a dissolvable cap (502) mounted to the distal tip (110), the dissolvable cap (502) covering the fixation tines (106) in a non-deployed state, the dissolvable cap (502) composed of a biodegradable material configured to dissolve and expose the fixation tines (106), achieving the deployed state, after a designated period of time in contact with biological fluid within the patient.

15. The IMD of any one of claims 1 to 9, wherein the deployment mechanism (108, 500) includes a dissolvable cap (502) mounted to the distal tip (110), an electrical contact (504) at a proximal end (506) of the housing (102), and a wire (508) that extends from the electrical contact (504) to the dissolvable cap (502), the dissolvable cap (502) covering the fixation tines (106) in a non-deployed state of the fixation tines (106), wherein the electrical contact (504) is configured to electrically connect to an electrical element of a delivery assembly (400) and receive an electric current from the electrical element that is conveyed via the wire (508) to the dissolvable cap (502), the dissolvable cap (502) comprising a polymer material configured to dissolve and expose the fixation tines (106), achieving the deployed state of the fixation tines (106), in response to receiving the electric current.
